# EUROPEAN PATENT SPECIFICATION

(11) **EP 2 888 001 B1**
(45) Date of publication and mention of the grant of the patent: **14.09.2016**
(21) Application number: 13728263.8
(22) Date of filing: 13.05.2013
(51) Int. Cl.: A61N 1/372, A61B 5/00

(54) **LOCATION-BASED USER ACCESS TO AND PROGRAMMING OF AN IMPLANTABLE MEDICAL DEVICE**
ORTSBASIERTE BENUTZERZUGRIFF AUF UND PROGRAMMIERUNG VON EINER IMPLANTIERBAREN MEDIZINISCHEN VORRICHTUNG
ACCÈS D'UTILISATEUR ET PROGRAMMATION D'UN DISPOSITIF MEDICAL IMPLANTABLE BASÉS SUR LOCALISATION.

(30) Priority: 27.08.2012 US 201261693398 P; 27.08.2012 US 201261693402 P
(43) Date of publication of application: 01.07.2015
(73) Proprietor: Cardiac Pacemakers, Inc., St. Paul, MN 55112-5798 (US)
(72) Inventor: HOYME, Kenneth P., Plymouth, Minnesota 55446 (US); KALGREN, James, Lino Lakes, Minnesota 55014 (US); LALONDE, John, Lake Elmo, Minnesota 55042 (US)
(74) Representative: Peterreins Schley
(86) International application number: PCT/US2013/040736
(87) International publication number: WO 2014/035494

(56) References cited:
- WO-A1-2012/063154
- US-A- 6 083 248
- US-A1- 2006 287 693
- US-A1- 2009 063 187

## Description

### BACKGROUND

Medical devices include devices designed to be implanted into a patient. Some examples of these implantable medical devices (IMDs) include cardiac function management (CFM) devices such as implantable pacemakers, implantable cardioverter defibrillators (ICDs), cardiac resynchronization therapy devices
(CRTs), and devices that include a combination of such capabilities. The devices can be used to treat patients or subjects using electrical or other therapy or to aid a physician or caregiver in patient diagnosis through internal monitoring of a patient's condition. The devices may include one or more electrodes in communication with one or more sense amplifiers to monitor electrical heart activity within a patient, and often include one or more sensors to monitor one or more other internal patient parameters. Other examples of IMDs include implantable diagnostic devices, implantable drug delivery systems, or implantable devices with neural stimulation capability.

IMDs can be sophisticated devices that can provide many advanced functions. External devices, such as IMD programmers, can communicate with an IMD using wireless telemetry and can be used to set device parameters. IMDs can also provide diagnostic data from one or more physiologic sensors. The
programmers or other external devices IMDs can also be used to collect diagnostic data obtained by one or more physiologic sensors of the IMD. Programming IMDs has become more complicated as therapeutic and diagnostic features are added to these types of devices. Interfaces to interact with these devices can be difficult for someone who only occasionally has to access the device. Therefore, it is desirable to simplify interactions with these complicated devices.

Published U.S. patent application no. 2009/063187 A1 discloses a portable source medical device determines communication links of a network presently available to effect communications with a target component when the source medical device is at each of a multiplicity of geographical locations. A profile is generated comprising information about each available communication link and attributes associated with each available communication link for each geographical location. When the source medical device is at a particular geographical location, a profile associated with the particular geographical location is accessed and a network connection is established between the source medical device and the target component using a communication link associated with the particular profile. Medical information is transferred between the source medical device and the target component via the communication link associated with the particular profile.

### OVERVIEW

The present invention relates to an external device as set out in claim 1 and a method as set out in claim 13. Other embodiments are described in the dependent claims.

This section is intended to provide an overview of subject matter of the present patent application. It is not intended to provide an exclusive or exhaustive explanation of the invention. The detailed description is included to provide further information about the present patent application.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, the various examples discussed in the present document.
FIG. 1 is an illustration of an example of portions of a system that includes an IMD.
FIG. 2 is an illustration representing programming an IMD at different locations.
FIG. 3 is a flow diagram of a method of providing location-based medical device services to a user.
FIG. 4 is a block diagram of an example of portions of an external device for communication with an implantable device.
FIG. 5 is a flow diagram of an example of a method of providing varying medical device services based on location and a type of user.
FIG. 6 is a block diagram of portions of an example of an external device for communication with an implantable device.
FIG. 7 is flow diagram of an example of a method for operating an external device that communicates with an implantable device.

### DETAILED DESCRIPTION

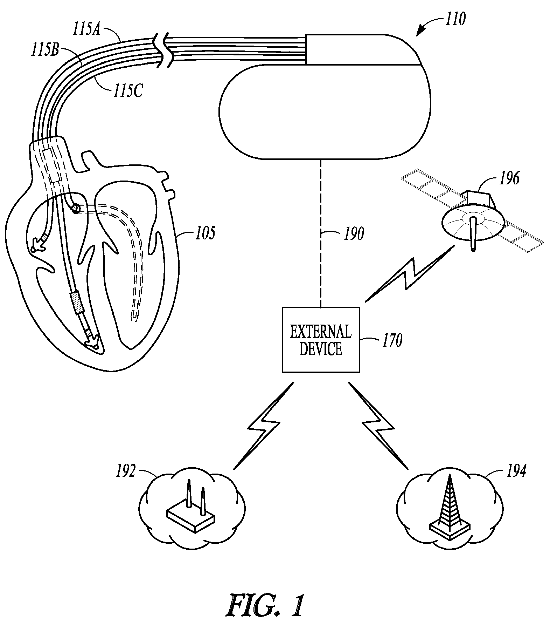

FIG. 1 is an illustration of an example of portions of a system that includes an IMD 110. Examples of IMD 110 include, without limitation, a pacemaker, a defibrillator, a cardiac resynchronization therapy (CRT) device, or a combination of such devices. The system 100 also typically includes an external device 170, such as an IMD programmer, that communicates wireless signals 190 with the IMD 110, such as by using radio frequency (RF) or other telemetry signals.

The IMD 110 can be coupled by one or more leads 115A-C to heart 105. Cardiac leads 115A-C include a proximal end that is coupled to IMD 110 and a distal end, coupled by electrical contacts or "electrodes" to one or more portions of a heart 105. The electrodes typically deliver cardioversion, defibrillation, pacing, or resynchronization therapy, or combinations thereof to at least one chamber of the heart 105. The electrodes may be electrically coupled to sense amplifiers to sense electrical cardiac signals. Sensed electrical cardiac signals can be sampled to create an electrogram. An electrogram can be analyzed by the IMD and/or can be stored in the IMD 110 and later communicated to the external device 170 where the sampled signals can be displayed for analysis.

As explained previously herein, programming of IMDs can be complicated and interfaces to interact with these devices can be difficult for someone who only occasionally has to access the device. The functions needed to be performed by an external device can be tied to location. Therefore, user interaction with the IMD can be simplified if the user interface only includes those functions tied to a given location.

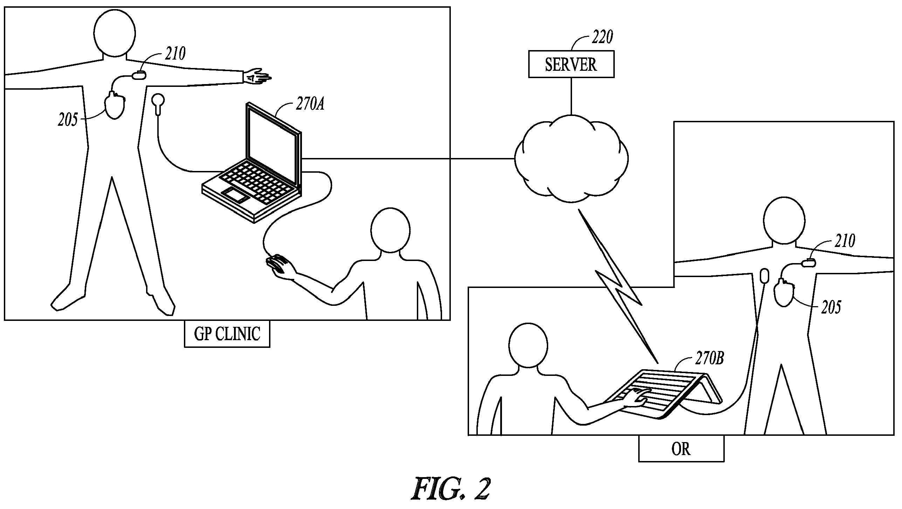

FIG. 2 is an illustration representing programming an IMD at different locations. One of the locations may be a general practice (GP) clinic. An external device 270A, 270B is used to program an IMD 210. Functionality at the general practice clinic may only require that certain information be read from the IMD 210 (e.g., for the physician or for uploading to one or more servers 220 remote from the general practice clinic), while functionality at a cardiologist's office may require full programming capability. The services required at the general practice clinic can be much more limited than the services required at the cardiologist's office and the user interface for the external device 270A, 270B can therefore be greatly simplified when the external device is located at the general practice clinic. In another example, the location is an operating room (OR). The IMD 210 may be an ICD. The required functionality of the user interface of the external device 270A, 270B may only be to turn defibrillation detection and therapy in the IMD 210 off and on.

It can be seen that the capabilities provided by the external device 270 can be changed based on the location of the device. If the external device 270A, 270B is able to determine its location, the user interface of the external device 270A, 270B can be automatically tailored and often simplified to meet the requirements of the location.

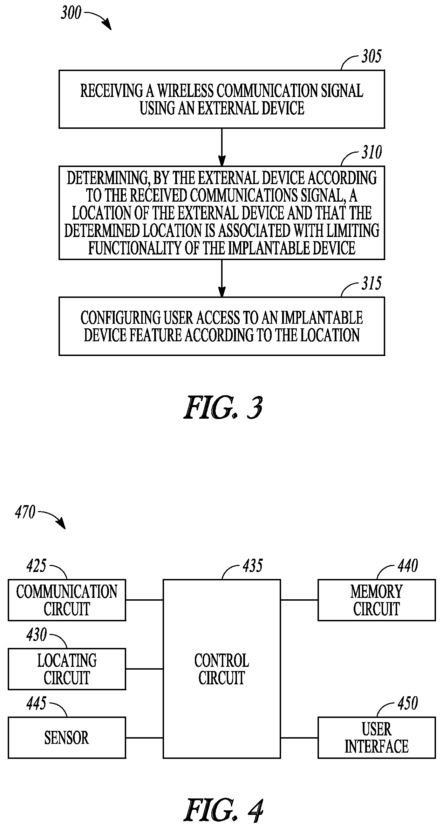

FIG. 3 is a flow diagram of a method 300 of providing location-based medical device services to a user. At block 305, a wireless communication signal is received using an external device. The external device can communicate with an implantable device (e.g., an IMD) and receive the wireless communication signal from at least one other device separate from the implantable device. As shown in FIG. 1, the wireless communication signal can be received from, among other things, a local area network (LAN) 192, a cellular telephone network 194, and a global positioning system (GPS) satellite 196. The wireless communication signal may be received using a communication circuit different from a telemetry circuit or telemetry system used for communication between the external device and the implantable device.

At block 310, the location of the external device is determined by the external device according to the received communication signal. The external device also determines whether the location is associated with limiting functionality of the implantable device. At block 315, user access to implantable device features is configured by the external device according to the determined location.

FIG. 4 is a block diagram of an example of portions of an external device 470 for communication with an implantable device. The external device 470 includes a communication circuit 425, a locating circuit 430, and a control circuit 435 that is electrically coupled to the communication circuit 425 and the locating circuit 430. In some examples, the communication circuit 425 includes a radio frequency (RF) receiver or transceiver. The communication circuit 425 receives a communication signal from at least one other device different from the implantable device, and the locating circuit 430 determines a location of the external device 470 using the received communication signal. The external device 470 may communicate with an implantable device using the communication circuit 425, or the external device 470 may include a separate telemetry circuit (not shown) for communication with the implantable device.

The control circuit 435 can include a microprocessor, a digital signal processor, application specific integrated circuit (ASIC), or other type of processor, interpreting or executing instructions in software modules or firmware modules. The control circuit 435 can include other circuits or sub-circuits to perform the functions described. These circuits may include software, hardware, firmware or any combination thereof. Multiple functions can be performed in one or more of the circuits or sub-circuits as desired.

The control circuit 435 determines whether the determined location imposes a limit on functionality of an implantable device and provides user access to one or more implantable device features according to the determined location. For example, the control circuit 435 may determine that the location of the external device is an OR, an emergency room (ER), a magnetic resonance imaging (MRI)
clinic, a catheterization lab, a cardiology clinic, an electrophysiology clinic, a hospital, or a general practice clinic, and configure (e.g., selectively provide or limit) user access to implantable device features accordingly.

The locating circuit 430 can include one or more of software, firmware, and hardware to perform the functions described herein. In certain examples, the locating circuit 430 is integral to the control circuit 435. In some examples, a tablet computer or a cellular telephone is integrated into the external device 470, and the locating circuit 430 is included in the tablet computer or cellular telephone.

In some examples, the locating circuit 430 determines the location of the external device 470 using global positioning (e.g., GPS, or assisted GPS). The communication signal may include one or more messages transmitted from satellites and the locating circuit 430 may determine the location of the external device 470 using the satellite transmission.

The locating circuit 430 may cross reference coordinates determined by global positioning with map information to determine that the coordinates are related to a hospital location or to a type of clinic for example. The map
information may be stored in a memory circuit 440 integral to, or electrically coupled to, the control circuit 435, or the map information may be stored in a remote server (e.g., a cloud server or cloud computer) accessed by the control circuit 435 using the communication circuit 425 or other port (e.g. a universal serial bus) to access the Internet.

In some examples, the locating circuit 430 determines the location of the external device 470 using cellular phone tower (e.g., cell site) information. The communication circuit 425 may receive a communication signal transmitted from a cell tower. The received communication signal may identify the cell tower transmitting the signal and the locating circuit 430 determines the location of the external device 470 from the cell tower identification. The locating circuit 430 may cross reference the cell tower information with location information (e.g., using a lookup table) from the memory or from access to cloud computing resources to determine the location of the external device 470.

In some examples, the locating circuit 430 determines the location of the external device 470 using wireless fidelity (WiFi) network mapping. The communication circuit 425 may receive a communication signal transmitted by a wireless fidelity (WiFi) network. The received communication signal may identify the WiFi network the locating circuit 430 may include a cross reference of WiFi nodes or networks for one or more areas. The received communication signal may identify the WiFi node or network (e.g., using an internet protocol address), and the location can then be determined from the cross reference. In certain examples, the communication signal identifies a LAN and the locating circuit 430 identifies the location of the external device 470 from the identification of the LAN.

In some examples, the locating circuit 430 uses a combination of two or more methods to identify the location of the external device. WiFi network or LAN information may provide intra-building information to the locating circuit 430. This information can help to further identify a function being performed at a location initially identified with GPS or cell tower information.

For instance, the communication circuit 425 may receive both a cell tower signal and a signal from a LAN network. The locating circuit 430 may identify a more general area using the cell tower signal (e.g., to identify that the external device is in a hospital) and identify a more specific area using the LAN signal (e.g., identify that the external device 470 is located in an operating theater or OR).

In some examples, when a location of the external device 470 is determined, the locating circuit 430 may identify a communication network associated with the determined location. The control circuit 435 may store an identifier for the communication network in the memory circuit in association with the determined location in the memory circuit 440. The locating circuit 430 can then deduce the location the next time an identifying signal is received from the communication network.

In some examples, the locating circuit 430 detects physical activity to determine if the location is changing or to adjust the location determination. The external device 470 may include an activity sensor 445 electrically coupled to the locating circuit. Some examples of an activity sensor include an accelerometer and a tilt switch. The locating circuit 430 estimates the location of the external device 470 according to the location determined using the received communication signal and subsequent motion sensed using the activity sensor. This may be useful to detect a change in the intra-building location of the external device 470. As explained previously herein, the control circuit 435 configures user access to one or more implantable device features according to the determined location. The external device 470 may include a user interface 450 electrically coupled to the control circuit. The user interface 450 may include one or more of a display, a mouse, a keyboard, and a touch sensitive or multi-touch sensitive display screen.

Using the user interface 450, the control circuit 435 may present implantable device features as available to a user according to the determined location. For example, the features may be selectable via indications presented on one or more user interface menus. The control circuit 435 may exclude presentation by the user interface 450 of an implantable device feature limited by the determined location. Excluding a feature may include not presenting the feature on a displayed menu or ghosting the feature on the display to indicate that the feature is not available to the user.

For example, it may be desirable to suspend the delivery of defibrillation therapy to a patient who is in an ER of a hospital. In response to the locating circuit 430 determining that the location of the external device 470 is an ER, the control circuit 435 configures the user interface to allow the user to enable or disable delivery of defibrillation therapy by the implantable device. The control circuit 435 may also allow the user to change a tachyarrhythmia detection rate of the implantable device in order to make the implantable device less sensitive to detection of tachyarrhythmia.

In another example, it may be desirable to place the implantable device in an electrocautery mode when the patient is undergoing surgery. In electrocautery mode, defibrillation or cardioversion therapy is disabled if the implantable device is an ICD. If the implantable device provides pacing therapy, electrocautery mode may include providing asynchronous pacing. In asynchronous pacing, the pacing
pulses are delivered according to a timer and not according to sensed cardiac events. This prevents non-cardiac events (e.g., the electrocautery) that may be sensed by the implantable device from influencing the therapy being provided. In response to the locating circuit 430 determining that the location of the external device 470 is an OR, the control circuit 435 allows a user to initiate the electrocautery mode in the implantable device. In certain examples, the control circuit 435 prevents access to implantable device features other than electrocautery mode when determining that the external device is in an ER. In certain examples, the control circuit 435 allows some information stored in the implantable device to be read or uploaded from the implantable device by the user.

In another example, it may be desirable to place the implantable device in an MRI mode when the patient is to undergo magnetic resonance imaging. Similar to the electrocautery mode, defibrillation or cardioversion therapy can be disabled in the MRI mode to avoid noise from the MRI causing false positive indications of the need for therapy. The MRI mode may also involve asynchronous pacing, and the asynchronous pacing may be delivered at a rate that is higher than a determined intrinsic rate. Pacing at a rate higher than the intrinsic rate prevents the implantable device from pacing into an intrinsic depolarization due to the presence of noise.

In response to the locating circuit 430 determining that the location of the external device 470 is an MRI clinic, the control circuit 435 configures the user interface 450 to allow a user to enable an MRI mode in the implantable device. In some examples, the control circuit 435 configures the user interface 450 to allow a user to initiate a device-automated pacing threshold test when it is determined that the external device 470 is located in an MRI clinic. In certain examples, the control circuit 435 does not allow user access to MRI mode or electrocautery mode, or these modes are disabled, unless the external device 470 is in an MRI clinic or OR, respectively.

For various locations, such as an OR, ER, or MRI clinic, it may be desirable to turn one or both of tachyarrhythmia detection and cardioversion/defibrillation therapy off. There is a concern that the user of the external device 470 may leave the detection or therapy turned off when the patient leaves the location. In some
examples, the control circuit 435 provides, on the user interface, an indication or alert as a reminder to turn one or both of the cardioversion/defibrillation therapy and tachyarrhythmia detection back on.

In some examples, the control circuit 435 presents a first alert to a user that delivery of defibrillation therapy is disabled and initiates a timer in the external device 470. The timer may be a timer circuit integral to, or electrically coupled to, the control circuit 435. The control circuit 435 may generate a second alert, upon timeout of the timer, regarding the disabled defibrillation therapy or disabled tachyarrhythmia detection.

In some examples, in response to the locating circuit 430 determining that the location of the external device 470 is a general practice clinic, the control circuit 435 configures the user interface to allow user read-only access to at least a portion of data or other information stored in the implantable device.

The control circuit 435 may initiate an action by the external device, without participation by a user, upon determining the location of the external device. For instance, the control circuit 435 may initiate communication of information to a remote server in response to the locating circuit 430 determining a location of the external device. The information to be communicated can be selected according to the determined location. In some examples, the remote server to receive the information is identified according to the determined location. For instance, the determined location may be a major medical center (e.g., Mayo Clinic). The control circuit 435 may initiate a transfer of information to a remote server associated with the medical center in response to determining that the external device 470 is located at the medical center. The selected information may be communicated to the remote server absent participation or prompting by a user. This allows specified
information to be made readily accessible to those people with the appropriate training. In some examples, emergency information can be transferred to a remote server and actions to address the emergency can be automatically dispatched.

The selected information may include location information and identification information of the external device. In this way, a field clinical engineer (FCE) or other device expert may be able to determine the locations of all units on a given
site (e.g., a hospital). This information may be relayed to a handheld device (e.g., a smart phone) of the FCE. Alerts from external devices may also be relayed to such handheld devices.

For some locations, the control circuit 435 allows user access to substantially all of the features of the implantable medical device. For instance, if the locating circuit 430 determines that the external device is located in the office of a cardiologist, a cardiology clinic, or an electrophysiology clinic, the control circuit 435 may allow user access to all programmable device parameters. Some device capability, such as changing executable code in the implantable device may still not be allowed by the control circuit 435.

Other levels of functionality may be desired for other locations. For instance, if the locating circuit 430 determines that the external device is located in a catheterization lab, the control circuit 435 may turn cardioversion or defibrillation therapy off and allow user access to pacing therapy capture detection tests.

In some examples, the control circuit 435 presents the user interface in a language appropriate to the detected location. For example, certain languages or language dialects can be associated with particular locations. The control circuit 435 may display user options in the language specified for the determined location.

In some examples, the external device 470 is dedicated to a certain location. In other words, it is intended that the external device 470 only be used at a specified location. When the locating circuit 435 determines that the location of the external device 470 is not at the specified location, the control circuit 435 may disable the external device 470 altogether (e.g., "brick" the device).

It can be seen from the examples described herein that because functions required to be performed by an external device are often dependent upon location, determining the location with the external device can lead to simplification of user interaction with an implantable device.

Additionally, access to features of a device located at the general practice clinic may be different for different personnel (e.g., a nurse at a general practice clinic or technician at an MRI clinic may be given access to a different subset of features than a doctor). Thus, the user interface can be even more simplified based on user role information. Access to features at a location such as a cardiologist's office may require role or other identity information to be entered into the external device to ensure the qualifications of the user. Thus, entering identity information into the external device can also provide security to the access given to IMD device features.

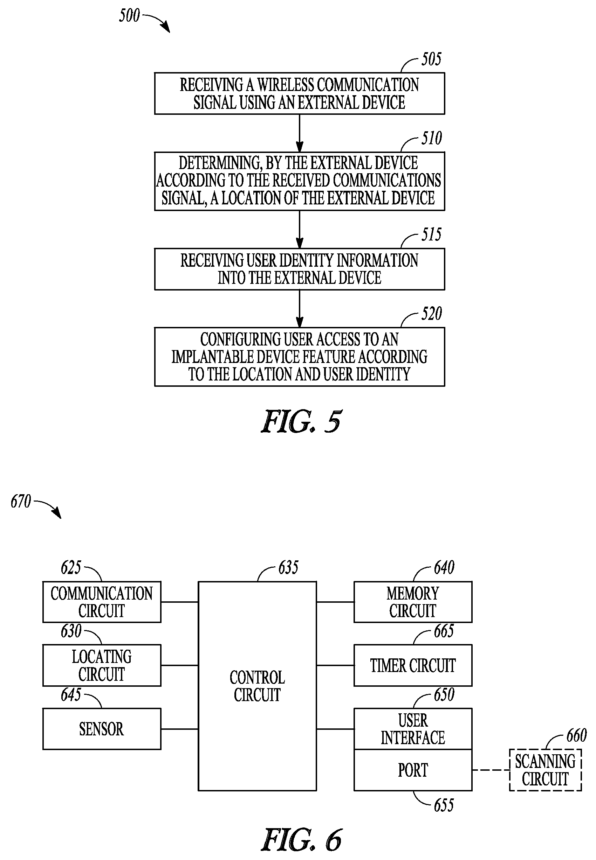

FIG. 5 is a flow diagram of an example of a method 500 of providing varying medical device services based on location and a type of user. At block 505, a wireless communication signal is received using an external device. The external device can communicate with an implantable device and receive the wireless communication signal from at least a third device separate from the implantable device using any of the wireless communication methods described herein.

At block 510, the location of the external device is determined by the external device according to the received communication signal. The external device may also determine whether the location is associated with limiting functionality of the implantable device.

At block 515, user identity information is received into the external device, such as by an input port. The external device may determine whether the identity information corresponds to a user role associated with limiting functionality of the implantable device. At block 520, user access to implantable device features is configured by the external device according to the determined location and the received user identity information.

FIG. 6 is a block diagram of portions of an example of an external device 670 for communication with an implantable device. The external device 670 includes a communication circuit 625, a locating circuit 630, a port 655, and a control circuit 635 that is electrically coupled to the communication circuit 625 and the locating circuit 630. The communication circuit 625 receives a communication signal from at least one other device different from the implantable device, and the locating circuit 630 determines a location of the external device 670 using the received communication signal. The external device can receive user identity information via the port 655.

The control circuit 635 allows user access to an implantable device feature according to the determined location and received user identity information. For example, the control circuit 635 may determine that the location of the external device is an OR, an ER, an MRI clinic, or a general practice clinic. Together with the user identification information, the control circuit 635 can configure (e.g., selectively provide or limit) user access to implantable device features according to the location. The locating circuit 630 may determine the location of the external device 670 using any of the locating method described herein.

The external device can receive the user identity information via the port 655, and the port 655 may include a communication port, or COMM port, to receive the user identity information. The COMM port may be a wired port or a wireless port. The COMM port may communicate using a standard protocol such as a USB protocol or firewire protocol.

In some examples, a user interface 650 is coupled to the port 655 to receive the user identity information. The user interface 650 may include one or more of a display, a mouse, a keyboard, and a touch sensitive or multi-touch sensitive display screen.

Using the port 655 to receive user identity information can also provide a level of security to accessing features of the implantable device. In some examples, the port 655 is coupled to a card reader that determines the user identity from an identification card of the user. The card reader may scan the identification card for information when the card is held before the card reader, or the identification card may be swiped through the card reader to provide the identity information. In some examples, the port 655 is coupled to a biometric scanner (e.g., a finger print scanner, a hand print scanner, etc.) and the port 655 receives biometric information of a user.

In some examples, a fourth device that is associated with the user identity information can be involved in obtaining the user identity information. The fourth device may be, among other things, a cellular telephone associated with the user, or a personal electronic device associated with the user that can access the internet. The communication circuit 625 may transmit access information receivable by the
fourth device according to a telephone number or an IP address associated with the user identity information.

The fourth device may retransmit the access information back to the external device 670 where it is received by the communication circuit 625. The control circuit 635 verifies that the received access information corresponds to the transmitted access information and allows user access to the implantable device feature or features according to the verification.

The access information can be machine readable. In some examples, the external device 670 includes a scanning circuit 660 that may be coupled to the port 655. The scanning circuit 660 may optically read the access information, such as by reading a barcode. The communication circuit 625 may transmit a barcode receivable by a telephone (e.g., a smartphone) associated with the user identity information. The scanning circuit 660 scans the barcode displayed on the telephone and the control circuit 635 verifies that the scanned barcode is the same as the transmitted barcode.

In some examples, the external device 670 includes a camera or other imaging device. The control circuit 635 initiates the taking of an image (e.g., a photograph) of the user who is given access to the external device 670. The image may be included in a record of access to the external device 670.

It can be seen from the examples described herein that because functions required to be performed by an external device are often dependent upon location, determining the location with the external device can lead to simplification of user interaction with an implantable device. Including user identification to determine the interface can simplify user interaction as well provide security in accessing the implantable device features.

As explained previously herein, the control circuit 635 configures user access to one or more implantable device features according to the determined location and user identity information. The control circuit 635 may present (e.g., display) implantable device features on a user interface 650 as available to a user according to the determined location and received user identity information. For example, the features may be selectable via indications presented on one or more user interface menus. The control circuit 635 may exclude presentation by the user interface 650 of an implantable device feature limited by one or both of the determined location and the user identity information. Excluding a feature may include not presenting the feature on a displayed menu or ghosting the feature on the display to indicate that the feature is not available to the user.

For example, it may be desirable to suspend the delivery of defibrillation therapy to a patient who is in an ER of a hospital. In response to the locating circuit 630 determining that the location of the external device 670 is an ER and receiving information that the user is a physician, the control circuit 635 configures the user interface 650 to allow the user to enable or disable delivery of defibrillation therapy by the implantable device. The control circuit 635 may also allow the user to change a tachyarrhythmia detection rate of the implantable device in order to make the implantable device less sensitive to detection of tachyarrhythmia.

In another example, it may be desirable to place the implantable device in an electrocautery mode when the patient is undergoing surgery. In electrocautery mode, defibrillation or cardioversion therapy is disabled if the implantable device is an ICD. If the implantable device provides pacing therapy, electrocautery mode may include providing asynchronous pacing. In asynchronous pacing, the pacing pulses are delivered according to a timer and not according to sensed cardiac events. This prevents non-cardiac events (e.g., the electrocautery) that may be sensed by the implantable device from influencing the therapy being provided. In response to the locating circuit 630 determining that the location of the external device 670 is an OR and based on the user identity information, the control circuit 635 allows an approved user to initiate the electrocautery mode in the implantable device.

In certain examples, the control circuit 635 prevents access to implantable device features other than electrocautery mode when determining that the external device is in an ER. In certain examples, the control circuit 635 prevents access to the electrocautery mode (e.g., does not provide the electrocautery mode in the displayed menu) according to the received user identity information. In certain examples, the control circuit 635 allows some information stored in the implantable device to be read or uploaded from the implantable device by the user based on the determined location and the user identity information.

In another example, it may be desirable to place the implantable device in an MRI mode when the patient is to undergo magnetic resonance imaging. Similar to the electrocautery mode, defibrillation or cardioversion therapy can be disabled in the MRI mode to avoid noise from the MRI causing false positive indications of the need for therapy. The MRI mode may also involve asynchronous pacing, and the asynchronous pacing may be delivered at a rate that is higher than a determined intrinsic rate. Pacing at a rate higher than the intrinsic rate prevents the implantable device from pacing into an intrinsic depolarization due to the presence of noise.

In response to the locating circuit 630 determining that the location of the external device 670 is an MRI clinic and based on the user identity, the control circuit 635 configures the user interface 650 to allow an approved user to enable an MRI mode in the implantable device. In some examples, the control circuit 635 configures the user interface 650 to allow the user to initiate a device-automated pacing threshold test when it is determined that the external device 670 is located in an MRI clinic. In certain examples, the control circuit 635 does not allow user access to MRI mode or electrocautery mode, or these modes are disabled, unless the external device 670 is in an MRI clinic or OR, respectively. In certain examples, the control circuit 635 does not allow user access to MRI mode or electrocautery mode when the identity information identifies the user as a technical support specialist rather than a physician.

In some examples, in response to the locating circuit 630 determining that the location of the external device 670 is a general practice clinic and the identity information identifying the user as a physician or nurse, the control circuit 635 configures the user interface 650 to allow user read-only access to at least a portion of data or other information stored in the implantable device.

In some examples, the control circuit 635 presents or displays features as available on the user interface according to the determined location, the received user identity and the type of implantable device. For instance, services related to cardioversion and defibrillation are only presented if the implantable device has one or both of cardioversion and defibrillation capability.

For some locations, the control circuit 635 allows user access to substantially all of the features of the implantable medical device. For instance, if the locating circuit 630 determines that the external device is located in the office of a cardiologist or a cardiology clinic and the control circuit 635 determines that the user is a cardiologist, the control circuit 635 may allow user access to all programmable device parameters. In another example, if the control circuit 635 determines that the user is a manufacturer's representative, the control circuit 635 may allow access to all features of the implantable device regardless of the location of the external device 670. Some device capability, such as changing executable code in the implantable device may still not be allowed by the control circuit 635.

When the control circuit 635 detects a change in location of the external device (e.g., as indicated by the locating circuit 630), the control circuit 635 can change user access to one or more implantable device features according to the changed location and identity information. For instance, control circuit 635 may change the user interface presented to a user when the location is changed from an office to an OR even though the user information remains unchanged.

For various locations, such as an OR, ER, or MRI clinic, it may be desirable to turn one or both of tachyarrhythmia detection and cardioversion/defibrillation therapy off. There is a concern that the user of the external device 670 may leave the detection or therapy turned off when the patient leaves the location.

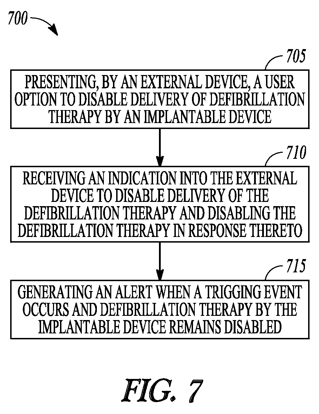

FIG. 7 is flow diagram of an example of a method 700 for operating an external device that communicates with an implantable device. At block 705, a user option is presented by the external device (e.g., using a user interface) to disable delivery of defibrillation therapy by the implantable device.

At block 710, an indication is received into the external device to disable delivery of the defibrillation therapy. The defibrillation therapy is disabled in the implantable device in response to receiving the indication. At block 715, an alert is generated when a triggering event occurs and defibrillation therapy by the implantable device remains disabled. The alert may be generated by the external
device or by a separate device that communicates the alert to the external device. The external device may then present the alert to the user.

Returning to the example of FIG. 6, the control circuit 635 can present a user option on the user interface 650 to disable delivery of one or both of defibrillation therapy and cardioversion therapy by the implantable device. The port 655 may receive an indication into the external device 670 to disable delivery of the defibrillation and/or cardioversion therapy. The control circuit 635 communicates a disable of the therapy to the implantable device in response to receiving the indication. The disable may be a device command communicated using wireless telemetry. In response to the control circuit 635 detecting a triggering event, the control circuit 635 presents an alert to a user (e.g., using the user interface 650) when the triggering event occurs and defibrillation therapy by the implantable device remains disabled. In some examples, the triggering event includes expiration of a timed duration. The external device 670 can include a timer circuit 665 electrically coupled to or integral to the control circuit 635. The control circuit 635 initiates timing of a specified (e.g., programmed) time duration by the timer circuit 665 in response to communication of the disable to the implantable device.

If the specified time duration expires and the defibrillation or cardioversion therapy by the implantable device remains disabled, the control circuit generates the alert and the alert is presented to the user. A complication to the approach can arise if the external device 670 is turned off or powered down. This could also turn off the timer circuit 665 and expiration of the timed duration would not be detected. In some examples, the control circuit 635 maintains circuit power to the timer circuit when the therapy is disabled until the defibrillation therapy by the implantable device is enabled or the alert is generated.

In some examples, the timing is done by a device different from the external device 670. In certain examples, the timing device is a server remote from the external device 670. The control circuit 635 may communicate an indication that delivery of defibrillation therapy is disabled to the remote server. When therapy is re-enabled using the external device 670, the control circuit 635 may communicate
an indication to the remote server that delivery of defibrillation therapy is re-enabled. The control circuit 635 receives the alert from the remote server when the remote server fails to receive the indication that the delivery of defibrillation therapy is re-enabled a specified time duration after receiving the indication that delivery of defibrillation therapy is disabled. The control circuit 635 then presents the alert to the user.

In some examples, the control circuit 635 transmits a status that
defibrillation/cardio version therapy is disabled to a remote server. The remote server may track multiple implantable devices whose therapy is disabled, and may time durations for each of the implantable devices. For example, the remote server may store a table in memory for the implantable devices. The table may include an indication of therapy status as "off when the therapy is disabled and therapy "on" when enabled or re-enabled. A timed duration can be initiated for any device whose status for one of the devices remains off when the timed duration for that device expires. The remote server may then send a reminder to a person, hospital, or clinic to re-enable the therapy.

In certain examples, the implantable device times the duration when the therapy is disabled. The control circuit 635 receives the alert from the implantable device when the control circuit fails to re-enable the delivery of defibrillation therapy after the specified time duration. The control circuit 635 then presents the alert to the user.

In certain examples, the control circuit 635 also transmits the alert that therapy was not re-enabled to a separate device such as a remote server. The remote server may send a reminder to a person, a hospital, or clinic to re-enable the therapy.

In some examples, the triggering event includes a change in location of the external device 670. According to the location or locations of the external device 670 determined by the locating circuit 630, the control circuit 635 generates the alert when determining that the location of the external device 670 has changed while defibrillation therapy by the implantable device remains disabled.

Detecting the disabling of one or both of defibrillation therapy and cardioversion therapy and providing a reminder to caregivers to re-enable the
therapy, can prevent inadvertent disabling by caregivers allowing a patient to receive the full benefit of the therapy provided by the implantable device.

The above detailed description includes references to the accompanying drawings, which form a part of the detailed description. The drawings show, by way of illustration, specific embodiments in which the invention can be practiced. These embodiments are also referred to herein as "examples."

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In the appended claims, the terms "including" and "in which" are used as the plain-English equivalents of the respective terms "comprising" and "wherein." Also, in the following claims, the terms "including" and "comprising" are open-ended, that is, a system, device, article, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

Method examples described herein can be machine or computer-implemented at least in part. Some examples can include a computer-readable medium or machine-readable medium encoded with instructions operable to configure an electronic device to perform methods as described in the above examples. An implementation of such methods can include code, such as microcode, assembly language code, a higher-level language code, or the like. Such code can include computer readable instructions for performing various methods. The code can form portions of computer program products. Further, the code can be tangibly stored on one or more volatile or non- volatile computer-readable media during execution or at other times. These computer-readable media can include, but are not limited to, hard disks, removable magnetic disks, removable optical disks (e.g., compact disks and digital video disks), magnetic cassettes, memory cards or sticks, random access memories (RAM's), read only memories (ROM's), and the like.

The above description is intended to be illustrative, and not restrictive. For example, the above-described examples (or one or more aspects thereof) may be used in combination with each other. Other embodiments can be used, such as by one of ordinary skill in the art upon reviewing the above description. The Abstract is provided to allow the reader to quickly ascertain the nature of the technical disclosure. It is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description, with each claim standing on its own as a separate embodiment. The scope of the invention should be determined with reference to the appended claims.

## Claims

1. An external device (170; 270A; 270B; 470; 670) for communication with an implantable device (105; 205), the external device comprising:
a communication circuit (425; 625) configured to receive a communication signal from at least one other device different from the implantable device (105; 205);
a locating circuit (430; 630) configured to determine a location of the external device (170; 270A; 270B; 470; 670) using the received communication signal; and
a control circuit (435; 635) electrically coupled to the communication circuit (425;
625) and the locating circuit (430; 630) and configured to:
provide user access to an implantable device (105; 205) feature according to the determined location, **characterised in that** the control circuit (435; 635) is further configured to determine whether the determined location imposes a limit on functionality of the implanted device.

2. The external device of claim 1, wherein control circuit (435; 635) is configured to determine that the location of the external device (170; 270A; 270B; 470; 670) is at least one of an operating room (OR), an emergency room (ER), a magnetic resonance imaging (MRI) clinic, a catheterization laboratory, a cardiology clinic, an electrophysiology clinic, a hospital, and a general practice clinic.

3. The external device of claim 1, wherein the locating circuit (430; 630) is configured to determine the location of the external device (170; 270A; 270B; 470; 670) using at least one of global positioning (196), wireless fidelity (WiFi) network mapping , cellular telephone tower identification, and local area network (LAN) identification.

4. The external device of claim 3, including:
an activity sensor (445; 645) electrically coupled to the locating circuit (430; 630),
wherein the locating circuit (430; 630) is configured to estimate the location of the external device (170; 270A; 270B; 470; 670) according to the location determined using the received
communication signal and subsequent motion sensed using the activity sensor (445; 645).

5. The external device of claim 1, including a user interface (450; 650) electrically coupled to the control circuit (435; 635), wherein the control circuit (435; 635) is configured to:
present, using the user interface (450; 650), the implantable device feature as available to a user according to the determined location; and
exclude presentation by the user interface (450; 650) of an implantable device feature limited by the determined location.

6. The external device of claim 5, wherein in response to the locating circuit (430; 630) determining that the location of the external device (170; 270A; 270B; 470; 670) is an ER, the control circuit (435; 635) is configured to allow, via the user interface (450; 650), a user to at least one of:
enable or disable delivery of defibrillation therapy by the implantable device (105; 205); and
change a tachyarrhythmia detection rate of the implantable device (105; 205).

7. The external device of claim 5,
wherein in response to the locating circuit (430; 630) determining that the location of the external device is an OR, the control circuit (435; 635) is configured to allow, via the user interface (450; 650), a user to initiate an electrocautery mode in the implantable device (105; 205), wherein defibrillation therapy is disabled in the electrocautery mode, and
wherein, when the electrocautery mode is initiated by a user, the control circuit (435; 635) is configured to:
present a first alert to a user that delivery of defibrillation therapy is disabled, initiate a timer in the external device (170; 270A; 270B; 470; 670); and
generate a second alert, upon timeout of the timer, regarding the disabled defibrillation therapy.

8. The external device of claim 5, wherein in response to the locating circuit (430; 630) determining that the location of the external device (170; 270A; 270B; 470; 670) is an MRI clinic, the control circuit (435; 635) is configured to allow, via the user interface (450; 650), a user to at least one of: enable an MRI mode in which cardiac pacing pulses are delivered asynchronously and at a rate higher than a determined intrinsic rate; and
initiate a device-automated pacing threshold test.

9. The external device of claim 5, wherein in response to the locating circuit (430; 630) determining that the location of the external device (170; 270A; 270B; 470; 670) is a general practice clinic, the control circuit is configured to allow, via the user interface (450; 650), user read-access to at least a portion of information stored in the implantable device (105; 205).

10. The external device of claim 5, wherein the control circuit (435; 635) is configured to display the user interface (450; 650) in a language specified for the determined location.

11. The external device of claim 1, including:
a memory circuit (440; 640) integral to or electrically coupled to the control circuit (435; 635), wherein the locating circuit (430; 630) is configured to identify a communication network associated with the determined location, and
wherein the control circuit (435; 635) is configured to store an identifier for the communication network in the memory circuit (440; 640) in association with the determined location.

12. The external device of any one of claims 1-11, wherein the control circuit (435; 635) is configured to:
selectively load information stored in the implantable device according to the determined location;
identify a remote server (220) according to the determined location; and communicate the information to the remote server (220) absent participation by a user.

13. The external device of any one claims 1-12, wherein the control circuit is configured to allow user access to an implantable device feature according to the determined location and received user identity information.

14. A method comprising:
receiving (305) a wireless communication signal using an external device , wherein
the external device is configured to communicate with an implantable device and receive the wireless communication signal from at least one other device;
determining (310), by the external device according to the received communication signal, a location of the external device ; and configuring (315), by the external device, user access to an implantable device feature according to the determined location, **characterised in that** the method further comprises determining (310), by the external device according to the received communication signal, that the determined location is associated with limiting functionality of the implantable device.

15. The method of claim 14, wherein the method comprises configuring (315), by the external device, user access to an implantable device feature according to the determined location and received user identity information.

## Patentansprüche

1. Externe Vorrichtung (170; 270A; 270B; 470; 670) für die Kommunikation mit einer implantierbaren Vorrichtung (105; 205), wobei die externe Vorrichtung umfasst:
eine Kommunikationsschaltung (425; 625), die dazu ausgelegt ist, ein Kommunikationssignal von zumindest einer anderen Vorrichtung als der implantierbaren Vorrichtung (105; 205) zu empfangen;
eine Ortungsschaltung (430; 630), die dazu ausgelegt ist, einen Ort der externen Vorrichtung (170; 270A; 270B; 470; 670) unter Verwendung des empfangenen Kommunikationssignals zu bestimmen; und
eine Steuerschaltung (435; 635), die elektrisch mir der Kommunikationsschaltung (425; 625) und der Ortungsschaltung (430; 630) gekoppelt ist und die ausgelegt ist dazu:
einem Benutzer Zugriff auf eine Funktion der implantierbaren Vorrichtung (105; 205) gemäß des bestimmten Orts bereitzustellen, **gekennzeichnet dadurch, dass** die Steuerschaltung (435; 635) weiter dazu ausgelegt ist, zu bestimmen, ob der bestimmte Ort eine Begrenzung einer Funktionalität der implantierbaren Vorrichtung auferlegt.

2. Externe Vorrichtung gemäß Anspruch 1, wobei die Steuerschaltung (435; 635) dazu ausgelegt ist, zu bestimmen, dass der Ort der externen Vorrichtung (170; 270A; 270B; 470; 670) mindestens einer ist von einem Operationssaal (OR), einer Notaufnahme (ER), einer Praxis für Kernspintomographie (MRI), einem Katheterlabor, einer kardiologische Praxis, einer elektrophysiologische Praxis, einem Krankenhaus oder einer allgemeinärztliche Praxis.

3. Externe Vorrichtung gemäß Anspruch 1, wobei die Ortungsschaltung (430; 630) dazu ausgelegt ist, den Ort der externen Vorrichtung (170; 270A; 270B; 470; 670) unter Verwendung zumindest einem von satellitengestützter Ortung (196), WLAN-(WIFI)-Netzwerk-Mapping, Identifizierung eines Mobilfunksendemasts, und LAN-Identifizierung zu bestimmen.

4. Externe Vorrichtung gemäß Anspruch 3, beinhaltend:
einen Aktivitätssensor (445; 645), der elektrisch mit der Ortungsschaltung (430; 630) gekoppelt ist, wobei die Ortungsschaltung (430; 630) dazu ausgelegt ist, den Ort der externen Vorrichtung (170; 270A; 270B; 470; 670) gemäß des Orts, der unter Verwendung des Kommunikationssignals bestimmt wird, und darauffolgender Bewegung, die durch den Aktivitätssensor (445; 645) detektiert wird, zu schätzen.

5. Externe Vorrichtung gemäß Anspruch 1, die eine Benutzerschnittstelle (450; 650), die elektrisch mit der Steuerschaltung (435; 635) gekoppelt ist, beinhaltet, wobei die Steuerschaltung (435; 635) ausgelegt ist zum:
Präsentieren, unter Verwendung der Benutzerschnittstelle (450; 650), der Funktion der implantierbaren Vorrichtung als verfügbar an einen Benutzer gemäß des bestimmten Orts; und
Ausschließen einer Präsentation durch die Benutzerschnittstelle (450; 650) einer Funktion der implantierbaren Vorrichtung, die durch den bestimmten Ort beschränkt wird.

6. Externe Vorrichtung gemäß Anspruch 5, wobei in Antwort darauf, dass die Ortungsschaltung (430; 630) bestimmt, dass der Ort der externen Vorrichtung (170; 270A; 270B; 470; 670) eine Notaufnahme ist, die Steuerschaltung (435; 635) dazu ausgelegt ist, dem Benutzer, über die Benutzerschnittstelle (450; 650) zumindest eines zu erlauben von:
Aktivieren oder Deaktivieren von der Zuführung von Defibrillations-Therapie durch die implantierbare Vorrichtung (105; 205); und
Ändern eine Rate zur Detektion von Tachykardie der implantierbaren Vorrichtung (105; 205).

7. Externe Vorrichtung gemäß Anspruch 5, wobei in Antwort darauf, dass die Ortungsschaltung (430; 630) bestimmt, dass der Ort der externen Vorrichtung (170; 270A; 270B; 470; 670) ein Operationssaal ist, die Steuerschaltung (435; 635) dazu ausgelegt ist, dem Benutzer über die Benutzerschnittstelle (450; 650) zu erlauben, einen Elektrokauterisations-Modus in der implantierbaren Vorrichtung (105; 205) zu initiieren, wobei Defibrillations-Therapie in dem Elektrokauterisations-Modus deaktiviert ist, und
wobei, wenn der Elektrokauterisations-Modus durch einen Benutzer initiiert wird, die Steuerschaltung (435; 635) dazu ausgelegt ist:
dem Benutzer einen ersten Alarm zu präsentieren, dass die Zuführung von Defibrillations-Therapie deaktiviert ist,
einen Zeitnehmer in der externen Vorrichtung (170; 270A; 270B; 470; 670) zu starten; und
nach einem Ablaufen des Zeitnehmers einen zweiten Alarm zu erzeugen in Bezug auf die deaktivierte Defibrillations-Therapie.

8. Externe Vorrichtung gemäß Anspruch 5, wobei in Antwort darauf, dass die Ortungsschaltung (430; 630) bestimmt, dass der Ort der externen Vorrichtung (170; 270A; 270B; 470; 670) eine MRI-Praxis ist, die Steuerschaltung (435; 635) dazu ausgelegt ist, dem Benutzer über die Benutzerschnittstelle (450; 650) zu erlauben, zumindest eines von:
Aktivieren eines MRI-Modus, in dem die Schrittmacher-Impulse asynchron zugeführt werden und mit einer Rate, die über einer bestimmten intrinsischen Rate liegt; und
Starten eines Vorrichtungs-automatisierten Schrittmachertherapie-Schwellwert-Tests.

9. Externe Vorrichtung gemäß Anspruch 5, wobei in Antwort darauf, dass die Ortungsschaltung (430; 630) bestimmt, dass der Ort der externen Vorrichtung (170; 270A; 270B; 470; 670) eine allgemeinärztliche Praxis ist, die Steuerschaltung (435; 635) dazu ausgelegt ist, dem Benutzer über die Benutzerschnittstelle (450; 650) zu erlauben, Lesezugriff auf zumindest einen Teil von Information zu haben, die in der implantierbaren Vorrichtung (105; 205) gespeichert ist.

10. Externe Vorrichtung gemäß Anspruch 5, wobei die Steuerschaltung (435; 635) dazu ausgelegt ist, die Benutzerschnittstelle (450; 650) in einer Sprache anzuzeigen, die für den bestimmten Ort spezifiziert wurde.

11. Externe Vorrichtung gemäß Anspruch 1, beinhaltend:
eine Speicherschaltung (440; 640), die integriert ist in oder elektrisch gekoppelt ist mit der Steuerschaltung (435; 635), wobei die Ortungsschaltung (430; 630) dazu ausgelegt ist, ein Kommunikationsnetzwerk, das mit dem bestimmten Ort assoziiert ist, zu erkennen, und
wobei die Steuerschaltung (435; 635) dazu ausgelegt ist, einen Identifikator für das Kommunikationsnetzwerk in der Speicherschaltung (440; 640) zu speichern, assoziiert mit dem bestimmten Ort.

12. Externe Vorrichtung gemäß irgendeinem der Ansprüche 1 bis 11, wobei die Steuerschaltung (435; 635) dazu ausgelegt ist:
selektiv Information, die in der implantierbaren Vorrichtung gespeichert ist, zu laden gemäß dem bestimmten Ort;
einen entfernten Server (220) gemäß dem bestimmten Ort zu identifizieren; und die Information an den entfernten Server (220) zu kommunizieren ohne Beteiligung eines Benutzers.

13. Externe Vorrichtung gemäß irgendeinem der Ansprüche 1 bis 12, wobei die Steuerschaltung (435; 635) dazu ausgelegt ist, einem Benutzer Zugriff auf eine Funktion der implantierbaren Vorrichtung zu geben gemäß dem bestimmten Ort und empfangener Information bezüglich einer Identität des Benutzers.

14. Verfahren, das umfasst:
Empfangen (305) eines drahtlosen Kommunikationssignals unter Benutzung einer externen Vorrichtung, wobei die externe Vorrichtung dazu ausgelegt ist, mit der implantierbaren Vorrichtung zu kommunizieren und das drahtlose Kommunikationssignal von zumindest einer anderen Vorrichtung als der implantierbare Vorrichtung zu empfangen;
Bestimmen (310), durch die externe Vorrichtung gemäß dem empfangenen Kommunikationssignal, eines Orts der externen Vorrichtung; und
Konfigurieren (315), durch die externe Vorrichtung, von Benutzer-Zugriff auf eine Funktion der implantierbaren Vorrichtung gemäß des bestimmten Orts,
**gekennzeichnet dadurch, dass** das Verfahren weiter Bestimmen (310), durch die externe Vorrichtung gemäß dem empfangenen Kommunikationssignal, das der bestimmte Ort assoziiert ist mit einer Beschränkung der Funktionalität der implantierbaren Vorrichtung, umfasst.

15. Verfahren gemäß Anspruch 14, wobei das Verfahren Konfigurieren (315), durch die externe Vorrichtung, von Benutzer-Zugriff auf eine Funktion der implantierbaren Vorrichtung gemäß des bestimmten Orts und empfangener Information bezüglich einer Identität des Benutzers umfasst.

## Revendications

1. Dispositif externe (170 ; 270A ; 270B ; 470 ; 670) destiné à la communication avec un dispositif implantable (105 ; 205), le dispositif externe comprenant :
un circuit de communication (425 ; 625) configuré pour recevoir un signal de communication de la part d'au moins un dispositif différent du dispositif implantable (105 ; 205) ;
un circuit de localisation (430 ; 630) configuré pour déterminer un emplacement du dispositif externe (170 ; 270A ; 270B ; 470 ; 670) en utilisant le signal de communication reçu ; et
un circuit de commande (435 ; 635) connecté électriquement au circuit de communication (425 ; 625) et au circuit de localisation (430 ; 630) et configuré pour :
fournir un accès par l'utilisateur à une fonctionnalité du dispositif implantable (105 ; 205) en fonction de l'emplacement déterminé, **caractérisé en ce que** le circuit de commande (435 ; 635) est en outre configuré pour déterminer si l'emplacement déterminé impose une limitation de la fonctionnalité du dispositif implanté.

2. Dispositif externe selon la revendication 1, le circuit de commande (435 ; 635) étant configuré pour déterminer que l'emplacement du dispositif externe (170 ; 270A ; 270B ; 470 ; 670) est au moins un parmi une salle d'opération (OR), une salle des urgences (ER), un cabinet d'imagerie par résonance magnétique (IRM), un laboratoire de cathétérisme, un cabinet de cardiologie, un cabinet d'électrophysiologie, un hôpital et un cabinet de médecine générale.

3. Dispositif externe selon la revendication 1, le circuit de localisation (430 ; 630) étant configuré pour déterminer l'emplacement du dispositif externe (170 ; 270A ; 270B ; 470 ; 670) en utilisant au moins un élément parmi le positionnement mondial (196), la cartographique d'un réseau d'accès sans fil (WiFi), l'identification d'un mât de téléphonie cellulaire et l'identification d'un réseau local (LAN).

4. Dispositif externe selon la revendication 3, comprenant un détecteur d'activité (445 ; 645) connecté électriquement au circuit de localisation (430 ; 630), le circuit de localisation (430 ; 630) étant configuré pour estimer l'emplacement du dispositif externe (170 ; 270A ; 270B ; 470 ; 670) en fonction de l'emplacement déterminé en utilisant le signal de communication reçu et un mouvement postérieur détecté en utilisant le détecteur d'activité (445 ; 645).

5. Dispositif externe selon la revendication 1, comprenant une interface d'utilisateur (450 ; 650) connectée électriquement au circuit de commande (435 ; 635), le circuit de commande (435 ; 635) étant configuré pour :
présenter, en utilisant l'interface d'utilisateur (450 ; 650), la fonctionnalité du dispositif implantable comme étant disponible à un utilisateur en fonction de l'emplacement déterminé ; et
exclure la présentation par l'interface d'utilisateur (450 ; 650) d'une fonctionnalité du dispositif implantable limitée par l'emplacement déterminé.

6. Dispositif externe selon la revendication 5, le circuit de commande (435 ; 635) étant configuré pour, en réponse à la détermination par le circuit de localisation (430 ; 630) que l'emplacement du dispositif externe (170 ; 270A ; 270B ; 470 ; 670) est une ER, autoriser, par le biais de l'interface d'utilisateur (450 ; 650), un utilisateur à effectuer au moins l'une des actions suivantes :
activer ou désactiver l'administration d'une thérapie de défibrillation par le dispositif implantable (105 ; 205) ; et
modifier une fréquence de détection de tachyarythmie du dispositif implantable (105 ; 205).

7. Dispositif externe selon la revendication 5,
le circuit de commande (435 ; 635) étant configuré pour, en réponse à la détermination par le circuit de localisation (430 ; 630) que l'emplacement du dispositif externe est une OR, autoriser, par le biais de l'interface d'utilisateur (450 ; 650), un utilisateur à initier un mode d'électrocautère dans le dispositif implantable (105 ; 205), la thérapie de défibrillation étant désactivée en mode d'électrocautère, et
lorsque le mode d'électrocautère est initié par un utilisateur, le circuit de commande (435 ; 635) étant configuré pour :
présenter une première alerte à un utilisateur lui signalant que l'administration de la thérapie de défibrillation est désactivée, initier une temporisation dans le dispositif externe (170 ; 270A ; 270B ; 470 ; 670) ; et
générer une deuxième alerte, à l'expiration de la temporisation, concernant la thérapie de défibrillation désactivée.

8. Dispositif externe selon la revendication 5, le circuit de commande (435 ; 635) étant configuré pour, en réponse à la détermination par le circuit de localisation (430 ; 630) que l'emplacement du dispositif externe (170 ; 270A ; 270B ; 470 ; 670) est un cabinet d'IRM, autoriser, par le biais de l'interface d'utilisateur (450 ; 650), un utilisateur à effectuer au moins l'une des actions suivantes :
activer un mode d'IRM dans lequel des impulsions de stimulation cardiaque sont délivrées de manière asynchrone à une fréquence supérieure à une fréquence intrinsèque déterminée ; et
initier un essai de seuil de stimulation automatisée par le dispositif.

9. Dispositif externe selon la revendication 5, le circuit de commande étant configuré pour, en réponse à la détermination par le circuit de localisation (430 ; 630) que l'emplacement du dispositif externe (170 ; 270A ; 270B ; 470 ; 670) est un cabinet de médecine générale, autoriser, par le biais de l'interface d'utilisateur (450 ; 650), un utilisateur à accéder en lecture à au moins une portion des informations stockées dans le dispositif implantable (105 ; 205).

10. Dispositif externe selon la revendication 5, le circuit de commande (435 ; 635) étant configuré pour afficher l'interface d'utilisateur (450 ; 650) dans une langue spécifiée pour l'emplacement déterminé.

11. Dispositif externe selon la revendication 1, comprenant :
un circuit de mémoire (440 ; 460) intégré au circuit de commande (435 ; 635) ou connecté électriquement à celui-ci, le circuit de localisation (430 ; 630) étant configuré pour identifier un réseau de communication associé à l'emplacement déterminé, et
le circuit de commande (435 ; 635) étant configuré pour stocker un identificateur pour le réseau de communication dans le circuit de mémoire (440 ; 460) en association avec l'emplacement déterminé.

12. Dispositif externe selon l'une quelconque des revendications 1 à 11, le circuit de commande (435 ; 635) étant configuré pour :
charger de manière sélective des informations stockées dans le dispositif implantable en fonction de l'emplacement déterminé ;
identifier un serveur distant (220) en fonction de l'emplacement déterminé ; et
communiquer les informations au serveur distant (220) sans participation d'un utilisateur.

13. Dispositif externe selon l'une quelconque des revendications 1 à 12, le circuit de commande étant configuré pour autoriser un accès par l'utilisateur à une fonctionnalité du dispositif implantable en fonction de l'emplacement déterminé et des informations d'identité d'utilisateur reçues.

14. Procédé comprenant :
réception (305) d'un signal de communication sans fil en utilisant un dispositif externe, le dispositif externe étant configuré pour communiquer avec un dispositif implantable et recevoir le signal de communication sans fil de la part d'au moins un autre dispositif ;
détermination (310), par le dispositif externe en fonction du signal de communication reçu, d'un emplacement du dispositif externe ; et
configuration (315), par le dispositif externe, de l'accès par l'utilisateur à une fonctionnalité du dispositif implantable en fonction de l'emplacement déterminé, **caractérisé en ce que** le procédé comprend en outre :
détermination (310), par le dispositif externe en fonction du signal de communication reçu, que l'emplacement déterminé est associé à une fonctionnalité de limitation du dispositif implantable.

15. Procédé selon la revendication 14, le procédé comprenant la configuration (315), par le dispositif externe, de l'accès par l'utilisateur à une fonctionnalité du dispositif implantable en fonction de l'emplacement déterminé et des informations d'identité d'utilisateur reçues.
